# EUROPÄISCHE PATENTSCHRIFT

(11) **EP 2 542 211 B1**
(45) Veröffentlichungstag und Bekanntmachung des Hinweises auf die Patenterteilung: **15.11.2017**
(21) Anmeldenummer: 11708403.8
(22) Anmeldetag: 03.03.2011
(51) Int. Cl.: A01N 25/04, A01N 25/30, A01N 43/40, A61Q 19/00, A61Q 19/10, A61K 9/107, A61K 31/14, A61K 31/155, A01N 47/44, A01N 37/12, A61K 8/37, A61K 8/41, A61K 9/00, A01N 33/12, A61K 8/49, A61Q 17/00

(54) **ANTIMIKROBIELLE ÖL-IN-WASSER-EMULSION ENTHALTEND QUATERNÄRE AMMONIUMVERBINDUNGEN**
ANTIMICROBIAL OIL-IN-WATER EMULSION CONTAINING QUATERNARY AMMONIUM COMPOUNDS
ÉMULSION HUILE DANS L'EAU ANTIMICROBIENNE CONTENANT DES COMPOSÉS AMMONIUM QUATERNAIRES

(30) Priorität: 03.03.2010 DE 102010010174
(43) Veröffentlichungstag der Anmeldung: 09.01.2013
(73) Patentinhaber: Bode Chemie GmbH, 22525 Hamburg (DE)
(72) Erfinder: MÜLLER, Kai-Martin, 22527 Hamburg (DE); PIETRYGA, Elke, 22047 Hamburg (DE)
(74) Vertreter: Kossak, Sabine
(86) Internationale Anmeldenummer: PCT/EP2011/001050
(87) Internationale Veröffentlichungsnummer: WO 2011/107281

(56) Entgegenhaltungen:
- WO-A2-2007/031520
- DE-A1- 4 405 510
- DE-A1-102008 020 797
- US-A1- 2006 051 385
- C. J. IOANNOU ET AL: "Action of Disinfectant Quaternary Ammonium Compounds against Staphylococcus aureus", ANTIMICROBIAL AGENTS AND CHEMOTHERAPY, Bd. 51, Nr. 1, 1. Januar 2007 (2007-01-01) , Seiten 296-306, XP55015889, ISSN: 0066-4804, DOI: 10.1128/AAC.00375-06
- REICH-SCHUPKE S ET AL: "Eradication of MRSA in chronic wounds of outpatients with leg ulcers is accelerated by antiseptic washes Results of a pilot study", INTERNATIONAL JOURNAL OF HYGIENE AND ENVIRONMENTAL HEALTH, URBAN U. FISCHER, JENA, DE, Bd. 213, Nr. 2, 1. März 2010 (2010-03-01), Seiten 88-92, XP026941891, ISSN: 1438-4639, DOI: 10.1016/J.IJHEH.2010.01.001 [gefunden am 2010-01-25]
- MADEO ET AL: "Efficacy of a novel antimicrobial solution (Prontoderm) in decolonising MRSA nasal carriage", JOURNAL OF HOSPITAL INFECTION, ACADEMIC PRESS, LONDON, GB, Bd. 74, Nr. 3, 1. März 2010 (2010-03-01), Seiten 290-291, XP026930977, ISSN: 0195-6701, DOI: 10.1016/J.JHIN.2009.09.008 [gefunden am 2009-11-14]

## Beschreibung

Gegenstand der Erfindung ist eine antimikrobielle Öl-in-Wasser-Emulsion, welche mindestens eine Ölkomponente, einen kationischen antimikrobiellen Wirkstoff, einen ersten nichtionischen Emulgator und Wasser, aber keine ionischen Emulgatoren, enthält, und die Verwendung dieser Öl-in-Wasser-Emulsion. Daneben ist Teil der Erfindung ein Verfahren zur Prävention der Besiedelung der Haut mit MRSA, sowie zur Prävention von Infektionen durch multi-resistente Keime mittels Sanierung der mit MRSA besiedelten Haut, welches das Aufbringen einer erfindungsgemäßen Öl-in-Wasser-Emulsion auf die Haut einer Person umfasst.

Die MRSA-Besiedelung stellt einen der häufigsten Auslöser von nosokomialen Infektionen dar. Unter MRSA, ursprünglich neben der Abkürzung ORSA ("Oxacillin-resistenter Staphylococcus aureus") nur eine Abkürzung für "Methicillinresistenter Staphylococcus aureus", versteht man solche Staphylococcus aureus Stämme, die gegen gegenwärtig verfügbare Beta-Lactam-Antibiotika eine Resistenz entwickelt haben. Häufig sind die MRSA multiresistent, d.h. sie zeigen auch gegenüber anderen Antibiotikaklassen eine Resistenz. Daneben gibt es auch noch VISA, VRSA und ORSA. VRSA (Vancomycin-resistenter Staphylococcus aureus) zeigen eine reduzierte Empfindlichkeit gegenüber Vancomycin, VISA (Vancomycin-intermediär-resistenter Staphylococcus aureus) besitzen hierüber hinaus eine geringere Empfindlichkeit gegenüber anderen Glycopeptid-Antibiotika.

An den Stellen des vorliegenden Textes, an denen von "MRSA" die Rede ist, ist, sofern kein gegenteiliger Hinweis gegeben ist, die Abkürzung "MRSA" stellvertretend für unterschiedliche multiresistente Keime gewählt.

MRSA werden am häufigsten dort übertragen, wo Menschen einen engen Kontakt haben und MRSA zusätzlich durch eine häufige Verwendung von Antibiotika einen besonderen Überlebensvorteil haben, d.h. vor allem in Krankenhäusern und Pflegeeinrichtungen. Resistent gewordene Keime können sich je nach Antibiotika-Typ trotz der Antibiotika-Therapie weiter vermehren.

Dabei sind nicht nur Patienten Träger von MRSA, sondern ansonsten auch vollkommen gesunde Personen können mit MRSA besiedelt sein. Bei einer Besiedelung sind die Stämme auf dem Körper sowie im Nasen-Rachen-Raum oder Darm anwesend; nur bei einer Infektion durch MRSA ist der Mensch durch die Keime krank. Da eine Besiedelung mit MRSA bei Gesunden häufig nicht bekannt ist, besteht ein besonderes Risiko, dass diese Träger die Keime an MRSAinfektionsgefährdete Personen weiterreichen und es dann zu einer schwer bis gar nicht bekämpfbaren Infektion kommt.

Von MRSA-Befall besonders betroffen sind Personen, die lange Zeiten im Krankenhaus verbringen müssen, etwa weil sie auf der Intensivstation liegen oder offene, schlecht oder nicht heilende Wunden haben. Auch andere abwehrgeschwächte Personen, etwa Krebs-Patienten, Patienten mit intravasalen Kathetern oder Dialyse- oder Beatmungspflicht, haben gegenüber gesunden Personen eine stärkere Gefährdung, über eine Besiedelung hinaus, eine MRSA-Infektion zu bekommen.

MRSA-infizierte Personen benötigen spezielle Antibiotika, gegen welche die MRSA bisher noch keine Resistenzen entwickelt haben. Zur Entfernung der MRSA bei diesen Gruppen, aber auch bei Personen, die lediglich von MRSA besiedelt sind, führt man bei Kenntnis eine MRSA-Sanierung durch, um eine weitere Übertragung der resistenten Keime zu verhindern, indem versucht wird, den Träger durch Verwendung von antibakteriellen und desinfizierenden Präparaten frei von MRSA zu bekommen. In Krankenhäusern werden nach speziellen Sanierungsplänen umfassende Sanierungsmaßnahmen durchgeführt, um eine Besiedelung zu bekämpfen, eine Beseitigung aller Keime zu erreichen und Neubesiedelungen zu verhindern.

Bei der MRSA-Sanierung ist allerdings häufig viel mehr gefordert als eine bloße Abtötung der Keime. Wenn die Haut geschädigt ist, so bedarf es mehr als einer Keimentfernung. Idealerweise muss gleichzeitig die geschädigte Haut mit Pflegestoffen versorgt werden.

Kern der herkömmlichen Sanierung ist eine antiseptische Ganzkörperwaschung, die über einen Zeitraum von mindestens 5 Tagen durchgeführt wird. Häufig erfolgt gleichzeitig eine Behandlung der Schleimhäute im Nasen-Rachen-Raum, etwa durch spezielle Nasensalben oder Lösungen zum Gurgeln. Um Rebesiedelungen zu vermeiden, ist nach den Waschungen frische Kleidung anzulegen, körpernahe Gegenstände, wie Brillen, sind zu reinigen, Bettwäsche zu wechseln.

Die Ganzkörperwaschung im Rahmen der Sanierung ist vorzugsweise für Patienten geeignet, die ihr Bett verlassen können. Bettlägerige Patienten erfordern sogenannte "leave-on"-Produkte, die z.B. mit Hilfe von Schwämmen oder Tüchern auf die Haut appliziert und dort belassen werden.

Aufgabe der vorliegenden Erfindung ist es daher, ein Mittel zur Prävention der Besiedelung der Haut mit MRSA und zur Sanierung MRSA-besiedelter Haut zu finden, das neben einer nachhaltigen Wirksamkeit gegen diverse multiresistente Keime eine gute Hautverträglichkeit mit hautpflegenden Eigenschaften zeigt.

Aus der Literatur sind diverse antimikrobielle Emulsionen oder Cremes bekannt, die zwar zur Händedesinfektion geeignet sind, jedoch keine Wirksamkeit gegenüber MRSA zeigen, wie etwa das Mittel gemäß JP 2007 284 412, welches Chlorhexidin oder Benzalkoniumchlorid enthält.

Aus US 638505 B1 ist eine antimikrobielle Lotion für die Haut mit Chlorhexidin, Benzalkoniumchlorid oder Biguanid als antimikrobiellen Wirkstoff bekannt, welche eine Langzeitwirkung über einen Zeitraum von 12 h zeigt. Die Lotion enthält zusätzlich ein Feuchthaltemittel, z.B. einen niederen Alkohol oder Glycerin, und einen Skin Conditioner, beispielsweise eine quaternäre Ammoniumverbindung.

US 6333039 B1 beschreibt eine opake desinfizierende und pflegende Zusammensetzung für die Haut, die als Wirkstoff einen niederen Alkohol in einer Konzentration von mindestens 50% enthält und außerdem Feuchthaltemittel, wie z.B. verschiedene Polymere.

Zur Behandlung unbelebter Oberflächen, insbesondere zur Reinigung harter Oberflächen und Entfernung fetthaltiger Rückstände, ist dem Fachmann aus US 6323171 B1 eine desinfizierende Mikroemulsion bekannt, welche als Wirkstoff quaternäre Ammoniumverbindungen enthält. Auch Alkohole sind als optionaler Bestandteil genannt. Eine Wirksamkeit gegen MRSA ist nicht erwähnt.

Aus dem Stand der Technik kennt der Fachmann also klassische antimikrobielle Wirkstoffe wie Chlorhexidin und Benzalkoniumchlorid. Auch antimikrobiell wirksame Zusammensetzungen auf alkoholischer Basis sind bekannt. Diese sind jedoch nicht zur Ganzkörperbehandlung geeignet, da Alkohole auf die Haut entfettend wirken und in empfindlichen Bereichen eine reizende Wirkung haben. Nachteilig an den Zusammensetzungen mit Alkohol als Wirkstoff ist zudem, dass Alkohol keine Remanenzwirkung hat.

Der Fachmann kennt auch aus dem Stand der Technik Substanzen, die eine Wirksamkeit gegen MRSA zeigen sollen, etwa PVP-lod in Cremes [P. Goldenheim, Postgraduate Medical Journal, Supplement (1993), 69(3), 62 - 65]. PVP-lod birgt jedoch ein zusätzliches Reizpotential für die Haut und die Schleimhäute, so dass die Behandlung der befallenen und ggf. bereits gereizten oder sogar offenen Haut kritisch zu beurteilen ist.

Ebenso ist dem Fachmann aus dem Stand der Technik bekannt, dass die Substanz Didecyldimethylammoniumchlorid eine Wirksamkeit gegen MRSA zeigt [C. loannou et al., Antimicro. Agents Chemother. 51, 2007, 296-306].

Die US 2006/0051385 A1 beschreibt Zusammensetzungen zur topischen Applikation zur Abtötung von Mikroorganismen. Dabei kann es sich um eine Öl-in-Wasser-Emulsion oder eine Wasser-in-Öl-Emulsion oder um Gele handeln. Als Emulgator wird eine katonische Verbindung eingesetzt.

DE 44 05 510 A1 beschreibt eine antimikrobielle Öl-in-Wasser-Emulsion, die mindestens eine Ölkomponente, einen ionischen Emulgator, z.B. eine quaternäre Ammoniumverbindung, einen nichtionischen Emulgator und Wasser enthält.

Der Fachmann kann dem Stand der Technik nicht entnehmen, welche Bestandteile ein Mittel enthalten müsste, das neben einer Wirksamkeit gegen MRSA auf belebten Flächen bei Geeignetheit zur Ganzkörperanwendung gleichzeitig eine hautpflegende Wirkung zeigt.
Die Aufgabe wird erfindungsgemäß gelöst durch eine antimikrobielle Öl-in-Wasser-Emulsion, enthaltend mindestens eine Ölkomponente, 0,01 bis 2 Gew.-% mindestens eines antimikrobiellen Wirkstoffes, dadurch gekennzeichnet, dass als antimikrobieller Wirkstoff Didecyldimethylammoniumchlorid eingesetzt wird,
0,1 bis 15 Gew.-% mindestens eines ersten nichtionischen Emulgators und Wasser, wobei die Emulsion ausschließlich nichtionische Emulgatoren und keine anionischen oder kationischen Emulgatoren enthält.
Weitere Ausführungsformen sind Gegenstand der Unteransprüche oder nachfolgend beschrieben.

Als Bestandteil der wässrigen Phase einer erfindungsgemäßen Öl-in-Wasser-Emulsion wird bevorzugt Wasser selbst eingesetzt. Ebenso können Gemische aus Wasser und Alkoholen eingesetzt werden, wobei der Alkoholbestandteil beispielsweise Ethanol, Propanol-1-ol, Propanol-2-ol oder auch ein aromatischer substituierter Alkohol, beispielsweise Phenoxyethanol oder Phenoxypropanol, oder auch eine Mischung mehrerer Alkohole, sein kann.

Als Ölkomponente dient mindestens ein Stoff, ausgewählt aus der Gruppe der Triglyceride bzw. Triglyceridester der gesättigten oder ungesättigten Fettsäuren oder Fettalkohole oder der verzweigten höheren Kohlenwasserstoffe, bevorzugt solche mit 13 bis 20 Kohlenstoffatomen. Beispielsweise können Capryl- oder Caprintriglycerid, Diethylhexylcarbonat, Cetylricinoleat oder C₁₃-C₁₄-Isoparaffin als Ölkomponente verwendet werden.

Die Zusammensetzung enthält 5 - 20 % einer Ölphase, vorzugsweise 10 - 20 % einer Ölphase, besonders bevorzugt 13 - 19 % einer Ölphase.

Die Ölphase enthält dabei bevorzugt mindestens zwei Ölkomponenten, von denen mindestens eine Ölkomponente bei Raumtemperatur flüssig und mindestens eine Ölkomponente bei Raumtemperatur fest vorliegt und weiter bevorzugt höchstens fünf Ölkomponenten. Die Zusammensetzung weist eine sehr hohe Viskosität auf und lässt sich dennoch sehr angenehm auf der Haut verteilen. Dieses ist zurückzuführen auf den Einsatz einer Ölphase mit mindestens zwei Ölkomponenten, die unterschiedliche Eigenschaften aufweisen.

Da eine erfindungsgemäße Öl-in-Wasser-Emulsion einen oder mehrere kationische Wirkstoffe enthält, sind zur Herstellung der Zusammensetzung anionische Verbindungen als Emulgatoren oder Verdicker nicht geeignet, da diese zu einer Agglomeration mit dem Wirkstoff neigen. Generell wirken ionische Verbindungen destabilisierend auf Emulsionen, so dass deren Formulierung Schwierigkeiten bereitet. Überraschenderweise konnte durch Verwendung lediglich nichtionischer Emulgatoren und einer geringen Konzentration des kationischen antimikrobiellen Wirkstoffes von maximal 2 Gew.-% eine Zusammensetzung erhalten werden, die nicht nur wirksam, sondern auch stabil ist.

Als erste nichtionische Emulgatoren, die neben der Funktion als Emulgator auch als Konsistenzgeber wirken können, werden erfindungsgemäß bevorzugt Glycerylmonoester, Glyceryldiester und Glyceryltriester von gesättigten oder ungesättigten, geradkettigen oder verzweigten C₆-C₂₂-Fettsäuren, bevorzugt Glycerylstearat, und/oder ethoxylierte gesättigte oder ungesättigte, geradekettige oder verzweigte C₆-C₂₂-Fettsäuren, bevorzugt PEG-100 Stearat, und/oder nicht-ethoxylierte gesättigte oder ungesättigte, geradkettige oder verzweigte C₆- C₂₂-Fettsäuren und/oder C₁₂-C₂₂-Fettalkohole, bevorzugt Stearylalkohol, und/oder ethoxylierte C₁₂-C₂₂-Fettalkohole eingesetzt.

Als hautpflegende Komponenten werden erfindungsgemäß solche eingesetzt, die als Feuchtigkeitsspender oder feuchtigkeitsbewahrend wirken, beispielsweise Glycerin, Propylenglycol oder Polyethylenglycol sowie synthetische Feuchtigkeitsspender wie Milchsäure oder ein Milchsäure-Derivat, z.B. Menthyllactat, Pyrrolidoncarbonsäure oder ein Pyrrolidoncarbonsäure-Derivat bzw. -Salz sowie ein pflanzlicher Feuchtigkeitsspender wie Aloe Vera-Bestandteile oder ein 1,2-Alkandiol oder Mischungen hiervon. Auch Triglyceride, z. B. Capryltriglycerid oder Caprintriglycerid, sind als Pflegekomponenten geeignet. Bevorzugt enthält die Öl-in-Wasser-Emulsion als Hautpflegekomponente Glycerin, Propylenglycol, Polyethylenglycol, Milchsäure, ein Milchsäurederivat, Pyrrolidoncarbonsäure, ein Salz oder Derivat der Pyrrolidoncarbonsäure, einen pflanzlichen Feuchtigkeitsspender und/oder ein 1,2-Alkandiol, bevorzugt in einer Menge von 0,1 bis 10 Gew.-%. Durch die Kombination der Ölkomponenten und Glycerin wird die Haut mit Fett und Feuchtigkeit versorgt.

Zur Viskositätseinstellung der ÖI-in-Wasser-Emulsion können übliche Verdickersubstanzen aus dem pharmazeutischen oder kosmetischen Bereich verwendet werden. Beispielsweise eignen sich Acrylsäurepolymere, Styrol-AcrylatCopolymere, Cellulosederivate, Guarderivate oder ethoxylierte Fettalkohole, insbesondere C₁₂-C₁₈-Fettalkohole, als Verdicker. Bevorzugte Verdickersubstanzen sind Polyacrylamid oder Laureth-7.

Bevorzugt weist die Öl-in-Wasser-Emulsion eine Viskosität von 10.000 bis 50.000 mPa*s bei 25°C auf, gemessen mittels eines Brookfield® DVII+ Rotationsviskosimeters mit einer RV6-Spindel bei einer Drehzahl von 10 U/min, einer Messzeit von 7,5 min und einer Wartezeit von 2 min. Das Messprinzip ist angelehnt an DIN 53018. Das Zeitintervall für die Messpunktaufnahme beträgt 15 s. Die Viskosität der Öl-in-Wasser-Emulsion beträgt vorzugsweise 21.000 - 47.000 mPa*s, besonders bevorzugt 31.000 - 45.000 mPa*s bei 25 °C.

Da die Emulsion thixotrope Eigenschaften, d.h. eine Abhängigkeit der Viskosität von der mechanischen Krafteinwirkung und deren Dauer, zeigt, ist bei den Messungen der Viskosität die Vorlaufzeit einzuhalten. Auch ist ein "Reifen" der Emulsion zu beobachten. Unmittelbar nach der Herstellung liegt die Viskosität der Emulsion zwar bevorzugt in genanntem Bereich, erhöht sich in den Tagen nach der Herstellung der Emulsion jedoch noch, um einen Wert in der Regel innerhalb dieses Bereiches zu erreichend, der auch längere Zeit, mindestens 3 Monate, stabil bleibt.

Aus dem hohen Ölanteil und den unterschiedlichen Eigenschaften der Ölkomponenten bei Raumtemperatur ergibt sich eine besondere Konsistenz der Zusammensetzung. Außerdem ergibt sich durch diese Eigenschaften ein thixotropes Verhalten. Das heißt, dass sich die Viskosität durch Scherung, wie zum Beispiel beim Verreiben zwischen den Händen, also beim Eincremen, ändert und abnimmt. Dadurch wird eine gute Verteilbarkeit und ein angenehmes Hautgefühl erreicht.

Um die Haltbarkeit einer erfindungsgemäßen Öl-in-Wasser-Emulsion zu erhöhen, ist es zweckmäßig, wenn die Öl-in-Wasser-Emulsion ein Konservierungsmittel enthält. Bevorzugt wird als Konservierungsmittel ein Alkohol verwendet, welcher 1 bis 4 Kohlenstoffatome aufweist. Besonders bevorzugt handelt es sich bei dem Alkohol als Konservierungsmittel um Propan-1-ol. Auch eine Verwendung von beispielsweise Propan-2-ol oder Ethanol oder eine Verwendung von Mischungen niederer Alkohole ist möglich. Geeignete Konservierungsmittel sind beispielsweise auch Phenoxyethanol und Phenoxypropanol. Bevorzugt ist das Konservierungsmittel in Konzentrationen von 0,01 bis 10 Gew.-%, besonders bevorzugt in einer Menge von 0,1 bis 5 Gew.-%, in der Öl-in-Wasser-Emulsion enthalten.

In einer bevorzugten Ausführungsform weist eine erfindungsgemäße Öl-in-Wasser-Emulsion die folgende Zusammensetzung auf: 5 bis 20 Gew.-% mindestens einer Ölkomponente, 0,01 bis 2 Gew.-%, bevorzugt 0,1 bis 1 Gew.-%, mindestens eines antimikrobiellen Wirkstoffes, 0,1 bis 10 Gew.-%, bevorzugt 0,5 bis 8 Gew.-%, besonders bevorzugt 1 bis 7 Gew.-%, ganz besonders bevorzugt 2 bis 5 Gew.-% mindestens eines nichtionischen Emulgators, 0,01 bis 10 Gew.-%, bevorzugt 0,1 bis 5 Gew.-%, mindestens eines Konservierungsmittels, bevorzugt eines einwertigen niederen Alkohols, 0,1 bis 10 Gew.-%, bevorzugt 2,5 bis 5 Gew.-% einer Hautpflegekomponente, 0,1 bis 5 Gew.-% eines Stabilisators und/oder Verdickers, 0,1 bis 5 Gew.-% eines zweiten nichtionischen Emulgators und 60 bis 85 Gew.-% Wasser, wobei sich die Komponenten zu 100 Gew.-% ergänzen.

Für die Auswahl des zweiten nichtionischen Emulgators gilt das bereits zu dem ersten nichtionischen Emulgator Gesagte.

In einer Ausführungsform weist die ÖI-in-Wasser-Emulsion einen pH-Wert von 5,5 bis 7,5 auf. Die Messung des pH-Wertes erfolgt unter Anwendung üblicher Methoden. Werte für erfindungsgemäße Öl-in-Wasser-Emulsionen, die in dem genannten Bereich lagen, wurden bei Messung der unverdünnten Zusammensetzung bei 21 °C mittels einer pH-Einstichelektrode Ingold LOT 406-M6-DXK-S7/25 erhalten.

Eine Möglichkeit zur Herstellung der erfindungsgemäßen ÖI-in-Wasser-Emulsion ist im folgenden beschrieben: Da die Komponenten der Ölphase teilweise bei Raumtemperatur im festen Zustand vorliegen, ist in der Regel zunächst ein Mischen der Komponenten in den entsprechenden Gewichtsanteilen erforderlich, gefolgt von einem Schmelzen. Die Schmelze wird zu der ebenfalls temperierten Wasserphase unter starkem Rühren zugegeben. Das Gemisch wird homogenisiert. Nach dem Abkühlen erfolgt eine Zugabe der weiteren Wirkstoffe und Hilfsstoffe. Zuletzt erfolgt die Zugabe des Verdickers, bevor das Gemisch erneut homogenisiert wird. Bei der Homogenisierung treten starke Scherkräfte auf, die einen erheblichen Einfluss auf die Verdickungsleistung und damit auf die Viskosität und die Tröpfchengröße der Emulsion haben.

Aufgrund der unterschiedlichen Aggregatzustände ist die Verarbeitung der Ölkomponenten in der Öl-in-Wasser-Emulsion besonders schwierig. Das feste Granulat, d.h. die bei Raumtemperatur feste Ölkomponente, muss in der Emulsion so fein verteilt sein, dass keine festen Partikel vorliegen, sondern eine "softe" Emulsion mit einer guten Verteilbarkeit erreicht wird. Dazu ist es notwendig, die gesamte Ölphase zu vereinen und aufzuschmelzen, damit sowohl die bei Raumtemperatur festen, als auch die bei Raumtemperatur flüssigen Ölkomponenten in flüssigem und somit mischbaren Zustand vorliegen. Die Ölphase und die Wasserphase, also Wasser und Glycerin werden zunächst auf 50 - 55 °C erwärmt und dann unter Rühren zusammengegeben. Die feine Struktur der Öl-in-Wasser-Emulsion wird nur durch starke Scherung bei dieser Temperatur erreicht. Das heißt, dass die Komponenten im Rührkessel nicht einfach nur gerührt werden, sondern zusätzlich mit einem Homogenisator bei hohen Scherraten miteinander vermischt werden.

Nach dem Abkühlen der Mischung werden Wirkstoffe, Konservierungsstoffe, sowie ggf. weitere Hilfsstoffe zugegeben und vermischt. Als letztes wird der Verdicker zugegeben und wiederum unter Rühren und zusätzlich unter Verwendung des Homogenisators bei hoher Scherrate in die Zubereitung gemischt. Die Geschwindigkeit beim Homogenisieren beträgt abhängig von der Reaktorgröße zum Beispiel 20 - 25 m/s. Die Reihenfolge der Prozessschritte ist dabei essentiell für die Gewährleistung einer homogenen Verteilung aller Inhaltsstoffe in einer stabilen Emulsion. Nach dem Abkühlen auf Raumtemperatur zeigt die erfindungsgemäße Emulsion besondere Eigenschaften bei der Anwendung, die auf die Verwendung der verschiedenen Stoffe in der Ölkomponenten zurück zu führen sind.

Erfindungsgemäße Öl-in-Wasser-Emulsionen eignen sich zur Herstellung von Pflege- und Desinfektionsmitteln zur Abtötung multi-resistenter Keime, bevorzugt zur Abtötung von methicillin-resistentem Staphylococcus aureus (MRSA), Oxacillin-resistentem Staphylococcus aureus (ORSA), Vancomycin-resistentem Staphylococcus aureus (VRSA), Vancomycin-intermediär-resistentem Staphylococcus aureus (VISA), Methicillin-resistentem Staphylococcus epidermis (MRSE), Vancomycin-resistentem Staphylococcus Epidermis (VRSE), Vancomycin-resistenten Enterokokken (VRE), Escherichia coli und Klebsiella pneumoniae. Außerdem haben sich erfindungsgemäße Öl-in-Wasser-Emulsionen wirksam gegen Proteus mirabilis, Proteus penneri, Samonella spp. und Enterobacter spp. gezeigt.

Die Erfindung betrifft insbesondere die Verwendung von erfindungsgemäßen Öl-in-Wasser-Emulsionen zur Abtötung multi-resistenter Keime auf der Haut, bevorzugt zur Abtötung von methicillin-resistentem Staphylococcus (Staph.) aureus (MRSA), Oxacillin-resistentem Staphylococcus aureus (ORSA), Vancomycin-resistentem Staphylococcus aureus (VRSA), Vancomycin-intermediär-resistentem Staphylococcus aureus (VISA), Methicillin-resistentem Staphylococcus epidermis (MRSE), Vancomycin-resistentem Staphylococcus Epidermis (VRSE), Vancomycin-resistenten Enterokokken (VRE), Escherichia coli und Klebsiella pneumoniae.

Eine erfindungsgemäße Öl-in-Wasser-Emulsion ist somit zur Prävention der Besiedelung der Haut mit multi-resistenten Keimen, sowie zur Prävention von Infektionen durch multi-resistente Keime, insbesondere durch die zuvor genannten Stämme, geeignet.

Erfindungsgemäße Öl-in-Wasser-Emulsionen dienen somit als Arzneimittel.

Die Erfindung umfasst damit auch ein Verfahren zur Prävention der Besiedelung der Haut mit MRSA, sowie zur Prävention von Infektionen durch multi-resistente Keime mittels Sanierung der mit MRSA besiedelten Haut, dadurch gekennzeichnet, dass eine erfindungsgemäße Öl-in-Wasser-Emulsion auf die Haut einer Person, insbesondere durch Aufträufeln, Aufstreichen, Einreiben, Aufspritzen, Aufsprühen oder durch Tauchen, aufgebracht wird.

Vorzugsweise werden 2 bis 6 g, insbesondere 3 bis 6 g einer erfindungsgemäßen Öl-in-Wasser-Emulsion pro m² Hautoberfläche auf die Haut der Person aufgetragen.

Bevorzugt führt eine erfindungsgemäße ÖI-in-Wasser-Emulsion in 60 min zu einer Reduktion der MRSA-Belastung um mindestens Reduktionsfaktor 5, gemessen, wie unter Beispiel 2 beschrieben, in Anlehnung an prEN 13727 (Entwurf August 2007).

Hier und im Folgenden bedeutet "unter Anlehnung an prEN 13727 (Entwurf August 2007)", dass die Prüfungen nach prEN 13727 (Entwurf August 2007) erfolgten, wobei Änderungen gegenüber der prEN 13727 (Entwurf August 2007) vorgenommen wurden, welche weiter unten beschrieben sind.

Die Pflege- und Desinfektionsmittel, enthaltend eine erfindungsgemäße Öl-in-Wasser-Emulsion, können als oder in Waschlotionen, Pflegelotionen oder als Bestandteil von "Leave-on"-Produkten, also solchen, die nach Applikation auf der Haut belassen werden, verwendet werden.

Ein aus der erfindungsgemäßen ÖI-in-Wasser-Emulsion hergestelltes Pflege- und Desinfektionsmittel kann mit herkömmlichen "rinse-off"-Produkten, d.h. insbesondere Waschlotionen, und "leave-on"-Produkten, beispielsweise Feuchttüchern, in der MRSA-Therapie kombiniert werden. Da die Lotion auf der Haut verbleibt, kann sie ihre antimikrobielle Wirksamkeit über einen langen Zeitraum von mehreren Stunden entfalten. Dabei wird gleichzeitig die Haut gepflegt, da sie durch die in der ÖI-in-Wasser-Emulsion enthaltenen hautfreundlichen Komponenten ideal versorgt wird. Es ist besonders vorteilhaft, wenn vor der Applikation der ÖI-in-Wasser-Emulsion eine Behandlung mit einer antimikrobiellen Waschlotion erfolgt.

MRSA tritt vermehrt im stationären und klinischen Bereich auf (Krankenhäuser und Pflegeheime) und wird (neben anderen Ursachen) dort oftmals aufgrund mangelnder Hygiene über die Hände des Pflegepersonals von einem Patienten zum nächsten übertragen. Im Falle eines planbaren Eingriffs wird entsprechend bei den Patienten ein Screening auf MRSA durchgeführt und vorher eine Sanierung veranlasst. Gerade bei älteren Patienten oder Bewohnern von Pflegeheimen kommt erschwerend hinzu, dass die Haut durch mangelnde Flüssigkeitsaufnahme eher zu einem trockenen Hautbild neigt. Die erfindungsgemäße Zusammensetzung ist daher als 2-in-1-Produkt anzusehen, das die antimikrobiellen Eigenschaften bezüglich MRSA mit einer hautfreundlichen und den Feuchtigkeitsgehalt der Haut erhaltenden bzw. verbessernden Eigenschaften in nicht vorhersehbarer Weise kombiniert.

Der erfindungsgemäßen Öl-in-Wasser-Emulsion wird in einem unabhängigen Gutachten eine signifikante Zunahme der Hautfeuchtigkeit um 38,7% nach einwöchiger Anwendung (einmal täglich) gegenüber der unbehandelten Situation zugeschrieben. Die Probanden beurteilten in einer subjektiven Befragung das Produkt im Hinblick auf die Anwendungseigenschaften. Dabei bewerteten 76 % der Probanden das Hautgefühl mit gut bis sehr gut, 81 % bewerteten die Verteilbarkeit mit gut bis sehr gut und 95 % die Verträglichkeit mit gut bis sehr gut.

### Beispiele

### Beispiel 1: Zusammensetzung einer erfindungsgemäßen Öl-in-Wasser-Emulsion

In Tabelle 1 ist die Zusammensetzung einer erfindungsgemäßen ÖI-in-Wasser-Emulsion angegeben.

**Tabelle 1**

| Inhaltsstoff | Gew.-% |
|---|---|
| Glycerin (85 Gew.-% in Wasser) | 3,47 |
| Glycerylstearat/ PEG-100 Stearat, Gewichtsverhältnis 1,67:1,00 (Tego Care 165) | 3,00 |
| Stearylalcohol | 0,75 |
| Caprin-/ Capryltriglycerid, Gewichtsverhältnis 1:1,86 (Tegosoft CT) | 7,50 |
| Diethylhexylcarbonat | 5,00 |
| Cetylrecinoleat | 2,00 |
| C₁₃-C₁₄-Isoparaffin | 0,30 |
| Polyacrylamid/ Laureth 7, Gewichtsverhältnis 1:7,5 (Sepigel 305) | 0,68 |
| Didecyldimethylammoniumchlorid (40 Gew.-% in Wasser) | 0,25 |
| Propan-1-ol | 4,00 |
| Wasser | 73,05 |

### Beispiel 2: Wirksamkeit einer erfindungsgemäßen ÖI-in-Wasser-Emulsion

Es wurde die antimikrobielle Wirksamkeit der Öl-in-Wasser-Emulsion gemäß Beispiel 1 gegenüber sechs nationalen MRSA-Stämmen untersucht (MRSA 134/93, MRSA 635/93, MRSA 994/93, MRSA 1000/93, MRSA 1150/93, MRSA 131/98).

Die bakterizide Wirkung der Emulsion im humanmedizinischen Bereich wurde durch eine modifizierte Verdünnungs-Neutralisationsmethode in Anlehnung an prEN 13727 (Entwurf August 2007) bestimmt. Es wurden nur Staphylococcus aureus-Stämme untersucht, und zwar die genannten Methicillin-resistenten Keime und lediglich zum Vergleich der durch die prEN 13727 (Entwurf August 2007) vorgegebene Methicillin-empfindliche Staphylococcus aureus Stamm ATCC 6538. Aufgrund dieser Abwandlung der prEN 13727 (Entwurf August 2007) war eine Prüfung der Wirksamkeit der Emulsion gegen MRSA möglich.

Hinsichtlich der Belastung wurden die Bedingungen für niedrige Belastung (siehe prEN 1327 (Entwurf August 2007) gewählt.

Hinsichtlich des Ansatzes der Proben wurde im Wesentlichen das in Absatz 5.5.4 der prEN 1327 (Entwurf August 2007) beschriebene Verfahren für ready-to-use Produkte angewandt. Hiervon abweichend wurde wegen der Konsistenz der Emulsion folgendermaßen verfahren:
9,7 g der ÖI-in-Wasser-Emulsion wurden in ein steriles Behältnis eingewogen. 0,2 ml der 5fach höher angesetzten (niedrigen) Belastung und 0,1 ml der 10fach höher konzentrierten Keimsuspension wurden hinzugegeben und gut durchmischt. Danach wurde sofort die Stoppuhr angestellt. Kurz vor Ablauf der Einwirkzeit (5 min, 15 min, 60 min) wurde das Gemisch durchgerührt und 1 g in das Neutralisationsmittel (8 ml) und Wasser (1 ml) eingewogen.
Das bei 121 °C und 15 min dampfsterilisierte Neutralisationsmittel wies folgende Rezeptur (Tabelle 2) auf:

**Tabelle 2**

| | |
|---|---|
| Pepton aus Casein, pankreatisch verdaut | 1,0 g |
| Natriumchlorid | 8,5 g |
| Tween 80 (Polysorbat 80) | 30,0 g |
| Lecithin | 3,0 g |
| L-Histidin | 1,0 g |
| Natriumthiosulfat, wasserfrei | 5,0 g |
| Saponin | 30,0 g |
| Ethersulfat, 70% | 14,29 g |
| Aqua bidest. | 1000 ml |

Die Bebrütungstemperatur betrug 37 ± 1 °C, die Prüftemperatur betrug 20 °C.

In Tabelle 3 sind die Wirksamkeiten der Öl-in-Wasser-Emulsion aus Beispiel 1 hinsichtlich der Keimreduktion bei den verschiedenen MRSA-Stämmen wiedergegeben. Dabei ist die Keimreduktion in logarithmischen Werten (Ig R) angegeben.

**Tabelle 3**

| | MRSA 134/93* | MRSA 635/93* | MRSA 994/93* | MRSA 1000/93* | MRSA 1150/93* | MRSA 131/98* |
|---|---|---|---|---|---|---|
| Einwirkzeit [min] | Ig R | Ig R | Ig R | Ig R | Ig R | Ig R |
| 5 | 4,30 | 4,62 | < 2,30 | < 2,38 | 2,98 | 4,78 |
| 15 | > 5,15 | > 5,50 | < 2,30 | 4,14 | 5,45 | > 5,44 |
| 60 | > 5,15 | > 5,50 | 5,04 | > 5,71 | > 5,66 | > 5,44 |

Gegen den Vergleichsstamm ATCC 33592 wurden Wirksamkeiten Ig R der Emulsion bei 5 min von 5,07, bei 6 min von > 5,53 und bei 60 min von > 5,53 festgestellt.

Die Prüfung nach prEN 13727 (Entwurf August 2007) gilt von einem Produkt als bestanden, wenn in einer validen Prüfung unter den Bedingungen für eine niedrige Belastung eine Keimreduktion um mindestens fünf logarithmische Stufen innerhalb von 60 min oder innerhalb eines kürzeren Zeitraums bei 20°C und der gewählten Belastungssubstanz nachgewiesen ist. Diese Wirksamkeit wurde gegen alle untersuchten MRSA-Stämme erreicht.

## Patentansprüche

1. Antimikrobielle Öl-in-Wasser-Emulsion, enthaltend
- mindestens eine Ölkomponente,
0,01 bis 2 Gew.-% mindestens eines antimikrobiellen Wirkstoffes, **dadurch gekennzeichnet, dass** als antimikrobieller Wirkstoff Didecyldimethylammoniumchlorid eingesetzt wird.
- 0,1 bis 15 Gew.-% mindestens eines ersten nichtionischen Emulgators und
- Wasser,
wobei die Emulsion ausschließlich nichtionische Emulgatoren und keine anionischen oder kationischen Emulgatoren enthält.

2. ÖI-in-Wasser-Emulsion gemäß Patentanspruch 1, **dadurch gekennzeichnet, dass** als erster nichtionisches Emulgator mindestens eine Verbindung, ausgewählt aus der Gruppe
Glycerylmonoester, Glyceryldiester und Glyceryltriester von gesättigten oder ungesättigten, geradkettigen oder verzweigten C₆ - C₂₂-Fettsäuren, bevorzugt Glycerylstearat, und/oder
ethoxylierte gesättigte oder ungesättigte, geradkettige oder verzweigte C₆ - C₂₂-Fettsäuren, bevorzugt PEG-100 Stearat, und/oder nicht-ethoxylierte gesättigte oder ungesättigte, geradkettige oder verzweigte C₆ - C₂₂-Fettsäuren und/oder C₁₂ - C₂₂-Fettalkohole, bevorzugt Stearylalkohol, und/oder ethoxylierte C₁₂ - C₂₂-Fettalkohole,
eingesetzt wird.

3. Öl-in-Wasser-Emulsion gemäß einem der vorhergehenden Patentansprüche, **dadurch gekennzeichnet, dass** die Emulsion ein Konservierungsmittel, bevorzugt 0,01 bis 10 Gew.-%, besonders bevorzugt 0,1 bis 5 Gew.-%, mindestens eines einwertigen niederen Alkohols, bevorzugt mit 1 bis 4 Kohlenstoffatomen, besonders bevorzugt Propan-1-ol, enthält.

4. Öl-in-Wasser-Emulsion gemäß einem der vorhergehenden Patentansprüche, **dadurch gekennzeichnet, dass** die Emulsion eine Hautpflegekomponente enthält, bevorzugt Glycerin, Propylenglycol, Polyethylenglycol, Milchsäure, ein Milchsäurederivat, Pyrrolidoncarbonsäure, ein Salz oder Derivat der Pyrrolidoncarbonsäure, einen pflanzlichen Feuchtigkeitsspender und/oder ein 1,2-Alkandiol oder Mischungen hiervon, bevorzugt in einer Menge von 0,1 bis 10 Gew.-%.

5. Öl-in-Wasser-Emulsion gemäß einem der vorhergehenden Patentansprüche, **dadurch gekennzeichnet, dass** die Ölkomponente mindestens einen Stoff umfasst, der bei Raumtemperatur fest ist und mindestens einen Stoff umfasst, der bei Raumtemperatur flüssig ist, wobei die Ölkomponente bevorzugt 2 bis 5 Stoffe umfasst.

6. Öl-in-Wasser-Emulsion gemäß einem der vorhergehenden Patentansprüche, **dadurch gekennzeichnet, dass** die Öl-in-Wasser-Emulsion besteht aus
- 5 bis 20 Gew.-% mindestens einer Ölkomponente,
- 0,01 bis 2 Gew.-%, bevorzugt 0,1 bis 1 Gew.-%, mindestens eines antimikrobiellen Wirkstoffes,
- 0,1 bis 10 Gew.-%, bevorzugt 0,5 bis 8 Gew.-%, besonders bevorzugt 1 bis 7 Gew.-%, ganz besonders bevorzugt 2 bis 5 Gew.-% mindestens eines ersten nichtionischen Emulgators,
- 0,01 bis 10 Gew.-%, bevorzugt 0,1 bis 5 Gew.-%, mindestens eines Konservierungsmittels, bevorzugt eines einwertigen niederen Alkohols,
- 0,1 bis 10 Gew.-%, bevorzugt 2,5 bis 5 Gew.-%, einer Hautpflegekomponente,
- 0,1 bis 5 Gew.-% eines Stabilisators und/oder Verdickers,
- 0,1 bis 5 Gew.-% eines zweiten nichtionischen Emulgators und
- 60 bis 85 Gew.-% Wasser,
wobei sich die Komponenten zu 100 Gew.-% ergänzen.

7. Öl-in-Wasser-Emulsion gemäß einem der vorhergehenden Patentansprüche, **dadurch gekennzeichnet, dass** die Emulsion einen pH-Wert von 5,5 bis 7,5 aufweist und/oder dass die Emulsion eine Viskosität von 10.000 - 50.000 mPa*s. vorzugsweise 21.000 - 47.000 mPa*s, besonders bevorzugt 31.000 - 45.000 mPa*s, bei 25°C aufweist, gemessen mittels eines Brookfield® DVII+ Rotationsviskosimeters mit einer RV6-Spindel bei einer Drehzahl von 10 U/min, einer Messzeit von 7,5 min und einer Wartezeit von 2 min.

8. Öl-in-Wasser-Emulsion gemäß einem der vorhergehenden Patentansprüche zur Verwendung bei der Abtötung von multi-resistenten Keimen, insbesondere von Methicillin-resistentem Staphylococcus aureus (MRSA), Oxacillin-resistentem Staph. aureus (ORSA), Vancomycin-resistentem Staph. aureus (VRSA), Vancomycin-intermediär-resistentem Staph. aureus (VISA), Methicillin-resistentem Staph. epidermis (MRSE), Vancomycin-resistentem Staph. Epidermis (VRSE), Vancomycin-resistenten Enterokokken (VRE), Escherichia coli und Klebsiella pneumoniae, auf der Haut.

9. Öl-in-Wasser-Emulsion gemäß einem der vorhergehenden Patentansprüche zur Verwendung bei der Reduktion der MRSA-Belastung um mindestens Reduktionsfaktor 5 in 60 min.

10. Pflege- und Desinfektionsmittel aus einer Öl-in-Wasser-Emulsion gemäß einem der vorhergehenden Patentansprüche 1 bis 7 zur Prävention der Besiedelung der Haut mit multiresistenten Keimen, sowie zur Prävention von Infektionen durch multi-resistente Keime mittels Sanierung der besiedelten Haut durch Abtötung multi-resistenter Keime, bevorzugt durch Abtötung von Methicillin-resistentem Staphylococcus aureus (MRSA), Oxacillin-resistentem Staph. aureus (ORSA), Vancomycin-resistentem Staph. aureus (VRSA), Vancomycin-intermediär-resistentem Staph. aureus (VISA), Methicillin-resistentem Staph. epidermis (MRSE), Vancomycin-resistentem Staph. Epidermis (VRSE), Vancomycin-resistenten Enterokokken (VRE), Escherichia coli und Klebsiella pneumoniae.

11. Pflege- und Desinfektionsmittel gemäß Anspruch 10, **dadurch gekennzeichnet, dass** das Mittel als Waschlotion, Pflegelotion oder als Bestandteil von Leave-on-Produkten verwendet wird.

12. Öl-in-Wasser-Emulsion nach einem der Ansprüche 1 bis 7 zur Verwendung als Mittel zur Prävention der Besiedelung der Haut mit multiresistenten Keimen, sowie zur Prävention von Infektionen durch multi-resistente Keime mittels Sanierung der besiedelten Haut durch Abtötung von multi-resistenten Keimen, insbesondere durch MRSA, wobei das Mittel geeignet ist, auf die Haut einer Person, insbesondere durch Aufträufeln, Aufstreichen, Einreiben, Aufspritzen, Aufsprühen oder durch Tauchen, aufgebracht zu werden.

13. Mittel nach Patentanspruch 12, **dadurch gekennzeichnet, dass** das Mittel geeignet ist zur Auftragung von 2 bis 6 g, insbesondere 3 bis 6 g der Öl-in-Wasser-Emulsion pro m² Hautoberfläche auf der Haut der Person.

## Claims

1. An antimicrobial oil-in- water emulsion containing:
- at least one oil component,
- 0.01 to 2 wt.% of at least one antimicrobial agent **characterized in that** didecyldimethylammonium chloride is used,
- 0.1 to 15 wt.% of at least one first nonionic emulsifier, and
- water
wherein the emulsion exclusively contains nonionic emulsifiers and no anionic or cationic emulsifiers.

2. The oil-in-water emulsifier according to Claim 1, **characterized in that** a compound selected from the group of
glyceryl monoesters, glyceryl diesters, and glyceryl triesters of saturated or unsaturated, straight-chain or branched C₆ - C₂₂ aliphatic acids, preferably glyceryl stearate, and/or
ethoxylated saturated or unsaturated, straight-chain or branched C₆ - C₂₂ aliphatic acids, preferably PEG-100 stearate, and/or
non-ethoxylated saturated or unsaturated, straight-chain or branched C₆ - C₂₂ aliphatic acids, and/or
C₁₂ - C₂₂ aliphatic alcohols, preferably stearyl alcohol, and/or
ethoxylated C₁₂ - C₂₂ aliphatic alcohols,
is used as the first nonionic emulsifier.

3. The oil-in-water emulsion according to one of the preceding claims, **characterized in that** the emulsion contains a preservative, preferably 0.01 to 10 wt.%, particularly preferably 0.1 to 5 wt.%, of at least one monohydric lower alcohol, preferably with 1 to 4 carbon atoms, particularly preferably 1-propanol.

4. The oil-in-water emulsion according to one of the preceding claims, **characterized in that** the emulsion contains a skin care component, preferably glycerin, propylene glycol, polyethylene glycol, lactic acid, a lactic acid derivative, pyrrolidone carboxylic acid, a salt or derivative of the pyrrolidone carboxylic acid, a vegetable moisturizer, and/or 1,2 alkanediol or mixtures of the same, preferably in an amount from 0.1 to 10 wt.%.

5. The oil-in-water emulsion according to one of the preceding claims, **characterized in that** the oil component comprises at least one substance that is solid at room temperature and at least one substance that is liquid at room temperature, wherein the oil component preferably comprises 2 to 5 substances.

6. The oil-in-water emulsion according to one of the preceding claims, **characterized in that** the oil-in-water emulsion comprises
- 5 to 20 wt.% of at least one oil component,
- 0.01 to 2 wt.%, preferably 0.1 to 1 wt.% of at least one antimicrobial agent,
- 0.1 to 10 wt.%, preferably 0.5 to 8 wt.%, particularly preferably 1 to 7 wt.%, more particularly preferably 2 to 5 wt.% of at least one nonionic emulsifier,
- 0.01 to 10 wt.%, preferably 0.1 to 5 wt.% of at least one preservative, preferably a monohydric lower alcohol,
- 0.1 to 10 wt.%, preferably 2.5 to 5 wt.% of a skin care component,
- 0.1 to 5 wt.% of a stabilizer and/or thickener,
- 0.1 to 5 wt.% of a second nonionic emulsifier, and
- 60 to 85 wt.% water
wherein the components add up to 100 wt.%.

7. The oil-in-water emulsion according to one of the preceding claims, **characterized in that** the emulsion has a pH value from 5.5 to 7.5, and/or that the emulsion has a viscosity from 10,000 - 50,000 mPa*s, preferably 21,000 - 47,000 mPa*s, particularly preferably 31,000 - 45,000 mPa*s at 25°C, measured by means of a Brookfield® DVII+ rotational viscometer using an RV6 spindle at a rotational speed of 10 rpm, a measuring period of 7.5 minutes, and a waiting period of 2 minutes.

8. The oil-in-water emulsion according to one of the preceding claims for use in killing multi-resistant bacteria, in particular, methicillin resistant Staphylococcus aureus (MRSA), oxacillin resistant Staphylococcus aureus (ORSA), vancomycin resistant Staphylococcus aureus (VRSA), vancomycin intermediate resistant Staphylococcus aureus (VISA), methicillin resistant Staphylococcus epidermis (MRSE), vancomycin resistant Staphylococcus epidermis (VRSE), vancomycin resistant enterococci (VRE), Escherichia coli, and klebsiella pneumoniae on the skin.

9. The oil-in-water emulsion according to one of the preceding claims for use in the reduction of the MRSA load by at least a reduction factor of 5 in 60 minutes.

10. A care and disinfecting agent made from an oil-in-water emulsion according to one of the preceding Claims 1 to 7 for preventing a colonization of the skin by multi-resistant bacteria, and for preventing infections by multi-resistant bacteria by means of sanitizing the colonized skin by killing multi-resistant bacteria, preferably by killing methicillin resistant Staphylococcus aureus (MRSA), oxacillin resistant Staphylococcus aureus (ORSA), vancomycin resistant Staphylococcus aureus (VRSA), vancomycin intermediate resistant Staphylococcus aureus (VISA), methicillin resistant Staphylococcus epidermis (MRSE), vancomycin resistant Staphylococcus epidermis (VRSE), vancomycin resistant enterococci (VRE), Escherichia coli, and klebsiella pneumoniae.

11. The care and disinfecting agent according to Claim 10, **characterized in that** the agent is used as a washing lotion, care lotion, or as a component of leave-on products.

12. The oil-in-water emulsion according to one of Claims 1 to 7 for use as an agent for preventing a colonization of the skin by multi-resistant bacteria, and for preventing infections by multi-resistant bacteria by means of sanitizing the colonized skin by killing multi-resistant bacteria, in particular MRSA, wherein the agent is suitable for being applied to the skin of a person, in particular by trickling on, brushing on, rubbing in, spraying on, or by immersion.

13. An agent according to Claim 12, **characterized in that** the agent is suitable for application on the skin of the person at 2 to 6 g, in particular 3 to 6 g of the oil-in-water emulsion per m² of skin surface.

## Revendications

1. Emulsion antimicrobienne de type huile-dans-eau, comprenant :
- au moins un composant huileux,
- de 0,01 à 2 % en poids d'au moins un agent antimicrobien, **caractérisée** en que l'on utilise, en tant qu'agent antimicrobien, du chlorure de didécyl-diméthyl-ammonium,
- de 0,1 à 15 % en poids d'au moins un premier émulsifiant non-ionique,
- et de l'eau,
laquelle émulsion contient exclusivement des émulsifiants non-ioniques et ne contient pas d'émulsifiants anioniques ou cationiques.

2. Emulsion de type huile-dans-eau, conforme à la revendication 1, **caractérisée en ce que** l'on utilise, en tant que premier émulsifiant non-ionique, au moins un composé choisi dans l'ensemble formé par les suivants :
- monoesters, diesters et triesters de glycérol et d'acide(s) gras en C₆-C₂₂ saturé(s) ou insaturé(s), à chaîne linéaire ou ramifiée, de préférence du stéarate de glycérol,
- et/ou acides gras en C₆-C₂₂ saturés ou insaturés, à chaîne linéaire ou ramifiée, éthoxylés, de préférence du stéarate de PEG-100,
- et/ou acides gras en C₆-C₂₂ saturés ou insaturés, à chaîne linéaire ou ramifiée, non éthoxylés,
- et/ou alcools gras en C₁₂-C₂₂, de préférence de l'alcool stéarylique, et/ou alcools gras en C₁₂-C₂₂ éthoxylés.

3. Emulsion de type huile-dans-eau, conforme à l'une des revendications précédentes, **caractérisée en ce que** l'émulsion contient un agent conservateur, de préférence de 0,01 à 10 % en poids et mieux encore de 0,1 à 5 % en poids, d'au moins un monoalcool inférieur, de préférence comportant de 1 à 4 atomes de carbone, et mieux encore de propane-1-ol.

4. Emulsion de type huile-dans-eau, conforme à l'une des revendications précédentes, **caractérisée en ce que** l'émulsion contient un composant de soin de la peau, de préférence du glycérol, du propy-lèneglycol, un polyéthylèneglycol, de l'acide lactique, un dérivé de l'acide lactique, de l'acide pyrrolidone-carboxylique, un sel ou un dérivé de l'acide pyrrolidone-carboxylique, un humidifiant végétal, et/ou un alcane-1,2-diol, ou un mélange de tels corps, de préférence en une proportion de 0,1 à 10 % en poids.

5. Emulsion de type huile-dans-eau, conforme à l'une des revendications précédentes, **caractérisée en ce que** le composant huileux comprend au moins une substance qui est solide à température ambiante et au moins une substance qui est liquide à température ambiante, étant entendu que le composant huileux comprend de préférence de 2 à 5 substances.

6. Emulsion de type huile-dans-eau, conforme à l'une des revendications précédentes, **caractérisée en ce que** l'émulsion de type huile-dans-eau est constituée :
- de 5 à 20 % en poids d'au moins un composant huileux,
- de 0,01 à 2 % en poids, et de préférence de 0,1 à 1 % en poids, d'au moins un agent antimicrobien,
- de 0,1 à 10 % en poids, de préférence de 0,5 à 8 % en poids, mieux encore de 1 à 7 % en poids, et surtout de 2 à 5 % en poids, d'au moins un premier émulsifiant non-ionique,
- de 0,01 à 10 % en poids, et de préférence de 0,1 à 5 % en poids, d'au moins un agent conservateur, de préférence un monoalcool inférieur,
- de 0,1 à 10 % en poids, et de préférence de 2,5 à 5 % en poids, d'un composant de soin de la peau,
- de 0,1 à 5 % en poids d'un stabilisant et/ou épaississant,
- de 0,1 à 5 % en poids d'un deuxième émulsifiant non-ionique,
- et de 60 à 85 % en poids d'eau,
étant entendu que le total de ces constituants fait 100 % en poids.

7. Emulsion de type huile-dans-eau, conforme à l'une des revendications précédentes, **caractérisée en ce que** l'émulsion présente un pH de 5,5 à 7,5 et/ou **en ce que** l'émulsion présente, à 25 °C, une viscosité de 10 000 à 50 000 mPa.s, de préférence de 21 000 à 47 000 mPa.s et mieux encore de 31 000 à 45 000 mPa.s, celle-ci étant mesurée au moyen d'un viscosimètre rotatif Brookfield® DVII+ muni d'une broche RV6, à une vitesse de 10 tours par minute, et avec une durée de mesure de 7,5 minutes et un temps d'attente de 2 minutes.

8. Emulsion de type huile-dans-eau, conforme à l'une des revendications précédentes, pour utilisation dans l'élimination, sur la peau, de germes multi-résistants, en particulier des suivants : Staphylococcus aureus résistant à la méthicilline (SARM), Staphylococcus aureus résistant à l'oxacilline (SARO), Staphylococcus aureus résistant à la vancomycine (SARV), Staphylococcus aureus résistant à un intermédiaire de vancomycine (SARIV), Staphylococcus epidermidis résistant à la méthicilline (SERM), Staphylococcus epidermidis résistant à la vancomycine (SERM), entérocoques résistants à la vancomycine (ERV), Escherichia coli et Klebsiella pneumoniae.

9. Emulsion de type huile-dans-eau, conforme à l'une des revendications précédentes, pour utilisation en vue d'une réduction de la charge en SARM d'un facteur d'au moins 5 en 60 minutes.

10. Produit de soin désinfectant, constitué d'une émulsion de type huile-dans-eau conforme à l'une des revendications 1 à 7, pour la prévention de la contamination de la peau par des germes multi-résistants, ainsi que pour la prévention, par désinfection de la peau contaminée, des infections causées par des germes multi-résistants, par élimination de germes multi-résistants, et de préférence par élimination de Staphylococcus aureus résistant à la méthicilline (SARM), de Staphylococcus aureus résistant à l'oxacilline (SARO), de Staphylococcus aureus résistant à la vancomycine (SARV), de Staphylococcus aureus résistant à un intermédiaire de vancomycine (SARIV), de Staphylococcus epidermidis résistant à la méthicilline (SERM), de Staphylococcus epidermidis résistant à la vancomycine (SERM), des entérocoques résistants à la vancomycine, d'Escherichia coli et de Klebsiella pneumoniae.

11. Produit de soin désinfectant, conforme à la revendication 10, **caractérisé en ce qu'**on utilise ce produit comme lotion de lavage, comme lotion de soin, ou comme constituant de produits sans rinçage.

12. Emulsion de type huile-dans-eau, conforme à l'une des revendications 1 à 7, pour utilisation en tant qu'agent pour la prévention de la contamination de la peau par des germes multi-résistants, ainsi que pour la prévention, par désinfection de la peau contaminée, des infections causées par des germes multi-résistants, par élimination de germes multi-résistants, en particulier de SARM, lequel agent est approprié pour qu'on puisse le déposer sur la peau d'une personne, en particulier en l'y versant goutte à goutte, en l'étalant ou en le pulvérisant dessus, en frictionnant ou en aspergeant celle-ci avec, ou en y faisant baigner celle-ci.

13. Agent conforme à la revendication 12, **caractérisé en ce que** cet agent est approprié pour le dépôt, sur la peau d'une personne, de 2 à 6 g et en particulier de 3 à 6 g de l'émulsion de type huile-dans-eau par mètre carré de surface de peau.
